(19) Europäisches Patentamt
European Patent Office
Office européen des brevets

(11) **EP 2 792 378 B1**

(12) **EUROPÄISCHE PATENTSCHRIFT**

(45) Veröffentlichungstag und Bekanntmachung des Hinweises auf die Patenterteilung:
**11.10.2017 Patentblatt 2017/41**

(21) Anmeldenummer: **14160867.9**

(22) Anmeldetag: **20.03.2014**

(51) Int Cl.:
*A61M 1/16* *(2006.01)*   *A61M 1/34* *(2006.01)*
*B01D 61/14* *(2006.01)*   *B01D 61/22* *(2006.01)*
*B01D 61/24* *(2006.01)*   *B01D 61/28* *(2006.01)*
*B01D 61/32* *(2006.01)*

(54) **Verfahren und Vorrichtung zur Ermittlung einer internen Filtration bei einer extrakorporalen Blutbehandlung**

Method and device for determining an internal filtration in extracorporeal blood treatment

Procédé et dispositif de détermination d'une filtration interne pour un traitement extracorporel du sang

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.04.2013 DE 102013103816**

(43) Veröffentlichungstag der Anmeldung:
**22.10.2014 Patentblatt 2014/43**

(73) Patentinhaber: **B. Braun Avitum AG**
**34212 Melsungen (DE)**

(72) Erfinder:
• **Wolff, Henrik**
  **37213 Witzenhausen (DE)**
• **Strohhöfer, Christof**
  **34123 Kassel (DE)**
• **Napierala, Roland**
  **33824 Werther (DE)**

(74) Vertreter: **Winter, Brandl, Fürniss, Hübner, Röss, Kaiser, Polte - Partnerschaft mbB Patent- und Rechtsanwaltskanzlei**
**Alois-Steinecker-Strasse 22**
**85354 Freising (DE)**

(56) Entgegenhaltungen:
**EP-A1- 1 867 353        WO-A1-2012/095066**
**DE-A1-102007 009 208    DE-T2- 60 026 530**
**US-A- 4 381 999**

**Beschreibung**

Die Erfindung betrifft eine Vorrichtung zum Reinigen von Blut. Eine Bestimmung einer internen Filtration bei einer extrakorporalen Blutbehandlung wie etwa einer Dialyse, das heißt einer Blutwäsche, ist möglich.

Aus der EP 0 240 101 A2 ist ein Dialysegerät mit einem Filter (Dialysator) bekannt, das eine Membran innerhalb einer abgedichteten Kammer aufweist. Am Einlass und Auslass des Filters ist jeweils eine Pumpe angeordnet, wobei die Blutströmungsrate anhand der Pumpendrehzahlen berechnet wird. Zudem wird eine der Pumpen so geregelt, dass eine bestimmte Abströmrate eingehalten und ein gewünschter durchschnittlicher Transmembran-Druck aufrechterhalten wird.

EP 1 867 353 A offenbart ein Hämodialysegerät, das einen arterienseitigen Blutkreislauf mit einer Blutpumpe, die mit einem Dialysator verbunden ist; und einen venenseitigen Blutkreislauf aufweist, der mit einer Vene verbunden ist. Eine Dialysat-Versorgungsleitung und eine Dialysat Entsorgungsleitung sind mit einer Seite des Dialysators verbunden. Ein Detektor dient zum Ermitteln eines Drucks im Blutkreislauf und ein weiterer Detektor zum Bestimmen eines Drucks in der Dialysatleitung. Ein Transmembrandruck wird auf der Grundlage der Drücke bestimmt. Wenn sich der Transmembrandruck um einen gegebenen Wert oder mehr erhöht, wird eine Umkehrfiltration durchgeführt, um ein Verstopfen der Poren einer Dialysemembran zu beseitigen.

Aus der US 4,381,999 ist ein System zum Zuführen von Fluid zu einem Dialysator bekannt, bei dem zum Erreichen einer Soll-Ultrafiltration eine automatische, periodische Überwachung der Ultrafiltrationsrate und des Transmembrandrucks durchgeführt wird. Der Transmembrandruck, der notwendig ist, um einen Sollflüssigkeitsverlust am Ende einer Sitzung zu erreichen, wird berechnet und der Transmembrandruck wird auf diesen berechneten Wert eingestellt.

DE 10 2007 009208 A1 offenbart eine Hohlfaser aus einem semipermeablen Membranmaterial, ein Faserbündel mit Hohlfasern, einen Filter mit einem Faserbündel sowie ein Verfahren zur Herstellung einer Hohlfaser oder eines Hohlfaserbündels. Generell ist die interne Filtration, die auch als interne Konvektion bezeichnet werden kann, außerhalb des Dialysators nicht messbar, jedoch von großem Interesse, um die konvektive Reinigungsleistung einer Blutbehandlung wie etwa einer Blutreinigungstherapie, z.B. einer Hämodialysetherapie zu quantifizieren.

Der Erfindung liegt die Aufgabe zugrunde, eine Vorrichtung zum Reinigen von Blut zu schaffen, die eine Ermittlung einer internen Konvektion bei einer Blutbehandlung ermöglicht und beispielsweise die interne Konvektion während der Behandlung regeln kann. Ein weiteres Ziel ist es, die ermittelte interne Konvektion vorzugsweise online dem Anwender anzuzeigen.

Mit der Erfindung wird eine Vorrichtung zum Reinigen von Blut gemäß Patentanspruch 1 bereitgestellt.

Ausgestaltungen der Erfindung sind in den Unteransprüchen angegeben.

Bei einem, mehreren oder allen Ausführungsbeispielen kann eine Anzeige der ermittelten internen Konvektion vorgesehen sein.

Bei der Vorrichtung kann zum extrakorporalen Reinigen des Bluts das Blut durch die als Filtereinrichtung dienende Reinigungseinrichtung, beispielsweise in Form eines Dialysators, geleitet und die interne Konvektion in der Reinigungseinrichtung z.B. auf der Grundlage von Druckunterschieden an der Reinigungseinrichtung ermittelt werden.

Eine Regeleinrichtung ist dazu ausgelegt, die ermittelte konvektive Filtration zu regeln.

Die Blutreinigung kann z.B. eine Hämodialyse oder eine Hämodiafiltration sein. Drucksensoren können zur Messung der Drücke des Bluts am Eingang und/oder am Ausgang der Reinigungseinrichtung vorgesehen sein. Optional können auch Drucksensoren zur Erfassung des Drucks einer Reinigungsflüssigkeit wie etwa einer Dialysierflüssigkeit am Eingang und/oder am Ausgang der Reinigungseinrichtung angeordnet sein. Alternativ hierzu können die Flüsse einer Reinigungsflüssigkeit und/oder des Blutes extrakorporal ermittelt und basierend hierauf rechnerisch oder anhand vorab hinterlegter, Dialysator spezifischer Werte/Kennlinien die Druckunterschiede bestimmt werden.

Zusätzlich kann eine Hämatokritbestimmung oder eine Ermittlung oder Vorgabe einer Plasmaviskosität oder einer Plasmaproteinkonzentration erfolgen. Es können auch zwei oder alle drei dieser Werte ermittelt werden.

Ferner wird ein mittlerer Transmembrandruck für die Filtration anhand von ermittelten Drücken berechnet und ein Druckkreuz aus dem Blutdruckverlauf und dem Reinigungsflüssigkeitsdruckverlauf, z.B. Dialysierflüssigkeitsdruckverlauf, ermittelt. Die interne Konvektion kann auf der Basis des Transmembrandrucks und gegebenenfalls zusätzlich unter Berücksichtigung eines Ultrafiltrationskoeffizienten als Produkt aus Filteroberfläche und Permeabilität berechnet werden. Die Filterkenngröße Transmembrandruck ist für interne Filtration nicht erforderlich, kann aber z.B. als Kontrollparameter zur Überwachung des Ultrafiltrationskoeffizienten KUFverwendet werden.

Die ermittelte interne Konvektion (konvektive Filtration) wird geregelt, vorzugsweise unter Vergleich eines ermittelten Werts der internen Filtration mit einem vorgegebenen Sollwert und durch Anpassung des Blutflusses des zu reinigenden Bluts und/oder des Flusses der Reinigungsflüssigkeit wie etwa der Dialysierflüssigkeit.

Die beanspruchte Vorrichtung zum Reinigen von Blut weist die Reinigungseinrichtung, vorzugsweise in Form eines Dialysators, die von dem Blut durchströmbar ist, und eine Ermittlungseinrichtung zur Ermittlung einer internen Konvektion in der Reinigungseinrichtung auf der Grundlage von Druckunterschieden an der Reinigungseinrichtung auf.

Die Vorrichtung kann zur Hämodialyse oder Hämodiafiltration ausgelegt und mit Drucksensoren zur Messung der Drücke des Bluts am Eingang und/oder am Ausgang der Reinigungseinrichtung versehen sein. Optional können ein oder mehrere Drucksensoren zur Erfassung des Drucks der Reinigungsflüssigkeit, z.B. einer Dialysierflüssigkeit am Eingang und/oder am Ausgang der Reinigungseinrichtung vorhanden sein.

Die Ermittlungseinrichtung kann zusätzlich zur Hämatokritbestimmung oder Vorgabe eines Hämatokrits ausgelegt sein, und/oder kann eine Ermittlung oder Vorgabe einer Plasmaviskosität oder einer Plasmaproteinkonzentration bewirken.

Die Vorrichtung ist dazu ausgelegt, einen mittleren Transmembrandruck für die Filtration anhand von ermittelten Drücken zu berechnen; ein Druckkreuz aus dem Blutdruckverlauf und dem Reinigungsflüssigkeitsdruckverlauf, vorzugsweise Dialysierflüssigkeitsdruckverlauf, zu ermitteln. Die interne Konvektion wird auf der Basis des Blutdruckverlaufs und des Reinigungsflüssigkeitsdruckverlaufs und/oder des Transmembrandrucks und gegebenenfalls zusätzlich unter Berücksichtigung eines Ultrafiltrationskoeffizienten als Produkt aus Filteroberfläche und Permeabilität berechnet.

Die Vorrichtung ist mit der Regeleinrichtung ausgestattet sein, die dazu ausgelegt ist, die ermittelte konvektive Filtration zu regeln, vorzugsweise unter Vergleich eines ermittelten Werts der internen Filtration mit einem vorgegebenen Sollwert und unter Anpassung des Blutflusses des zu reinigenden Bluts und/oder des Flusses der Reinigungsflüssigkeit wie etwa der Dialysierflüssigkeit.

Dies erlaubt eine Ermittlung der internen Filtration oder Konvektion innerhalb der Blutreinigungeinrichtung wie etwa eines Dialysators während der Durchführung der Blutreinigung, z.B. der Hämodialyse, also gewissermaßen online. Damit kann die konvektive Reinigungsleistung der Blutreinigung wie etwa der Hämodialysetherapie quantifiziert werden. Es ist damit eine Berechnung der internen Konvektion während der Behandlung möglich.

Weiterhin ist eine Regelung der internen Konvektion während der Behandlung erreichbar.

Bei einem oder mehreren Ausführungsbeispielen der Erfindung ist es möglich, beispielsweise die interne Filtration als aktuelle Flussrate sowie optional zusätzlich oder alternativ das bisherige Volumen der Filtration oder des gereinigten Blutes bzw. der Dialysierflüssigkeit und gegebenenfalls auch einen Erwartungswert dieses Volumens zum Ende der Therapie anzugeben. Hierbei ist auch eine Umrechnung in die Clearance, das heißt die erreichte Reinigung, möglich, falls gewünscht.

Diese Angabe der internen Filtration kann während der Durchführung der Blutreinigung wie etwa der Hämodialyse, also gewissermaßen online, erfolgen. Ferner ist es möglich, beispielsweise auf der Grundlage eines Messsignals während der laufenden Behandlung, also wiederum "online", eine Anpassung von Einflussparametern vorzunehmen, die sich auf die interne Konvektion bzw. Filtration auswirken. Diese Anpassung der Einflussparameter wie etwa der Strömungsrate des Blutes oder z.B. der Dialysierflüssigkeit, der Temperatur oder dergleichen kann beispielsweise durch eine entsprechende Anpassung des Blutflusses oder des Dialysierflüssigkeitsflusses und/oder auch über eine Druckveränderung auf der Blutseite, oder durch eine Veränderung der Dialysierflüssigkeitstemperatur durchgeführt werden. Durch die Druckverschiebung auf der Blutseite wird eine Phase aus der Filtration und anschließend aus einer Backfiltration, also Rückfiltration, erzeugt.

Weiterhin ist es

möglich, das aktuelle Volumen während der laufenden Behandlung und/oder die aktuelle Flussrate des Blutes beispielsweise auf einem Monitor oder einem Zeigerinstrument darzustellen oder in sonstiger optischer, akustischer oder anderer Form wiederzugeben.

Ausführungsbeispiele der erfindungsgemäßen Vorrichtung werden nachstehend anhand der Zeichnungen näher erläutert.

Fig. 1 zeigt eine schematische Anordnung eines Ausführungsbeispiels der erfindungsgemäßen Vorrichtung,
Fig. 2 veranschaulicht die über den Blutfluss aufgetragene interne Filtration bei einem Ausführungsbeispiel,
Fig. 3 zeigt eine Grafik für die interne Filtration in Abhängigkeit von dem Dialysierflüssigkeitsfluss,
Fig. 4 zeigt eine graphische Darstellung von Druckverläufen im Dialysator auf der Blutseite und der Dialysierflüssigkeitsseite bei einem Ausführungsbeispiel ohne Verwendung eines Hämatokritwerts,
Fig. 5 veranschaulicht Druckverläufe innerhalb des Dialysators für die Blutseite und Dialysierflüssigkeitsseite bei einem Ausführungsbeispiel mit Verwendung eines Hämatokritwerts
Fig. 6 illustriert einen Verlauf der internen Filtration über die Länge des Dialysators,
Fig. 7 zeigt eine graphische Darstellung eines möglichen Verlaufs eines internen filtrierten Volumens über die Therapiedauer, und
Fig. 8 veranschaulicht ein Ausführungsbeispiel einer erfindungsgemäßen Vorrichtung.

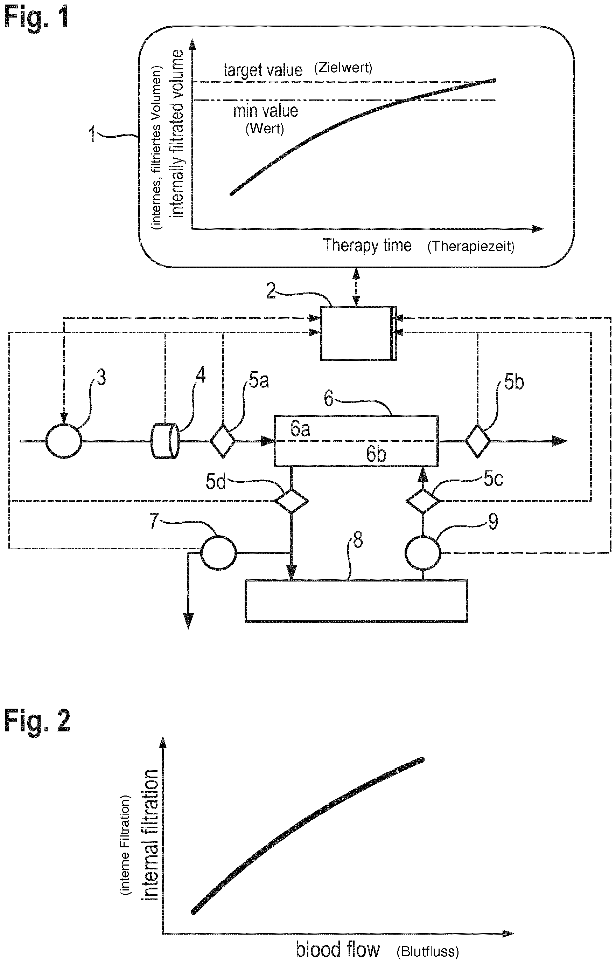

Fig. 1 zeigt ein Ausführungsbeispiel einer erfindungsgemäßen Vorrichtung (Gerät), die einen Dialysator 6 mit einer Blutkammer 6a und einer Dialysierflüssigkeitskammer 6b enthält. Das zu reinigende Blut wird über eine Blutpumpe 3 zugeführt und durch einen Sensor 4 zur Bestimmung eines Blutparameters wie etwa zur Hämatokritbestimmung zum Dialysator 6 geleitet. Drucksensoren 5a, 5b am Eingang und Ausgang des Dialysators 6 erfassen den Druck des Bluts am Eingang bzw. Ausgang des Dialysators.

In einem Bilanzsystem 8 wird eine Bilanzierung auf der Basis der Dialysierflüssigkeit durchgeführt, die über eine Dialysierflüssigkeitspumpe 9 zur Dialysierflüssigkeitskammer 6b gespeist wird. Der eingangsseitige Druck der Dialysierflüssigkeit am Eingang der Dialysierflüssigkeitskammer 6b wird durch einen Drucksensor 5c gemessen. Ein weiterer Drucksensor 5d erfasst den Druck der Dialysierflüssigkeit bei Austritt der Dialysierflüssigkeit aus der Dialysierflüssigkeitskammer 6b. Die aus der Dialysierflüssigkeitskammer 6b wieder ausgetretene Dialysierflüssigkeit wird dem Bilanzsystem 8 zugeführt und zusätzlich oder alternativ über eine Ultrafiltrationspumpe 7, zu einem Auslass- oder Abfallbehälter geleitet werden, wie dies durch den Pfeil veranschaulicht ist.

Ein Regler 2 erhält die aktuellen Werte aller Drücke und Flüsse und ggfls. weitere Blutparameter wie etwa einen ermittelten/eingegebenen Hämatokritwert, wie dies durch die gepunkteten Linien veranschaulicht ist. Der Regler 2 stellt bei Bedarf neue Werte für den Blutfluss und/oder den Dialysierflüssigkeitsfluss ein und steuert die Blutpumpe 3 und die Dialysierflüssigkeitspumpe 9 sowie die Ultrafiltrationspumpe 7 entsprechend. Eine Anzeige 1 zeigt beispielsweise die berechneten Parameter an, zum Beispiel in Form eines graphischen Schaubilds, wie dies in Fig. 1 im Block 1 veranschaulicht ist. Weiterhin kann an oder auf der Anzeige 1 auch die Eingabe von Vorgaben und Sollwerten für den Regler 2 durch entsprechende Tastenbetätigungen oder entsprechende Eingaben erfolgen. Die Anzeige 1 kann mit einem Speicher und/oder einem Prozessor ausgestattet oder mit diesem verbunden sein, der die entsprechenden Auswertungen berechnet und Sollgrößen für die Pumpensteuerung und andere Steuerungen berechnen kann.

Auf der Basis der Parameter, d.h. der Spezifikation, der jeweils eingesetzten Blutreinigungseinrichtung wie etwa des Dialysators 6 können die nachfolgenden Berechnungen durchgeführt werden. Zu diesen Parametern zählen die geometrischen Parameter wie etwa die Dialysatoroberfläche, das Volumen, die Faserlänge sowie Innendurchmesser, Außendurchmesser und Anzahl der Fasern usw. des Dialysators sowie die Permeabilität für die Filtration und die Backfiltration. Weiterhin sind die Kennlinien von Interesse, die einen Zusammenhang zwischen Blutfluss und Dialysierflüssigkeitsfluss einerseits und der erzeugten internen Konvektion andererseits herstellen.

In Fig. 2 ist schematisch eine graphische Darstellung einer Kennlinie aufgetragen, wobei auf der Abszisse der Blutfluss, das heißt die Blutströmung in ml/min, und auf der Ordinate die Größe der internen Filtration aufgetragen ist. Die Kennlinie verläuft im Wesentlichen linear mit abnehmender Steigung bei höherem Blutfluss. Die Kennlinie kann je nach jeweils eingesetztem Dialysator 6 selbstverständlich auch einen anderen, aber jeweils im Voraus bekannten Verlauf aufweisen.

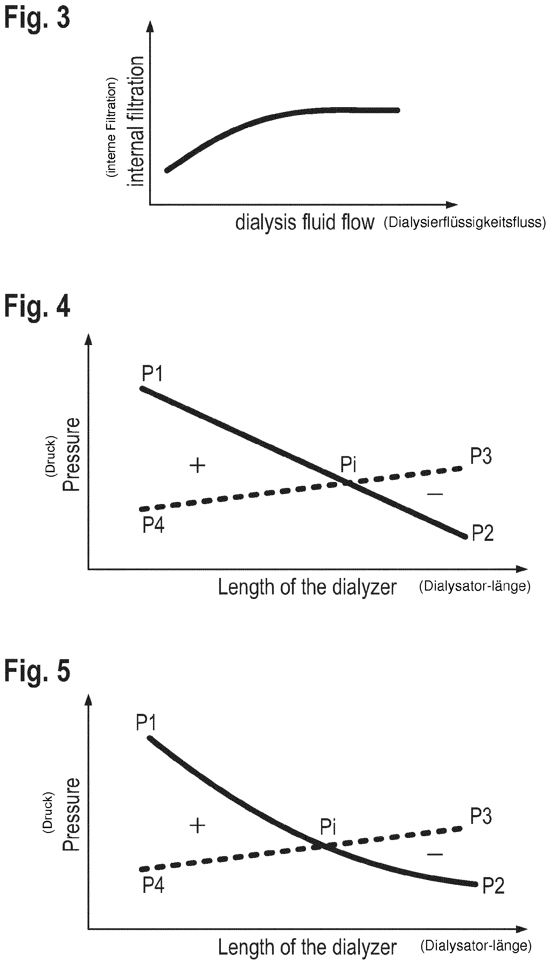

In Fig. 3 ist eine graphische Darstellung des Zusammenhangs zwischen dem Dialysierflüssigkeitsfluss (Abszisse) und der internen Filtration (Ordinate) dargestellt. Wie aus Fig. 3 ersichtlich ist, nimmt die interne Filtration mit zunehmender Dialysierflüssigkeitsströmung deutlich verlangsamt zu und nähert sich relativ rasch asymptotisch einem Grenzwert an.

Aus den Fign. 2 und 3 ist ersichtlich, dass die Abhängigkeit der internen Filtration vom Dialysierflüssigkeitsfluss ein sehr viel stärker nichtlineares Verhalten aufweist als die Abhängigkeit der Filtration vom Blutfluss (Fig. 2). Dieser Sachverhalt ist durch unterschiedliche Ursachen bedingt. In einem idealen System steigt der blutseitige Druckabfall linear mit dem Blutfluss an, was sich in einer linearen Steigerung der internen Filtration niederschlägt. Demgegenüber ruft ein verstärkter Filtrationsfluss über die Filtermembran interne Veränderungen hervor, so dass der Zusammenhang in der Realität von der reinen Linearität abweicht.

Erhöht man allerdings beispielsweise den Druckabfall auf der Dialysierflüssigkeitsseite durch einen gesteigerten Fluss der Dialysierflüssigkeit während man den Blutfluss konstant hält (entscheidend ist hier die Aufkonzentration nicht membrangängiger Substanzen wie Zellen oder Proteine), gelangt man in einen Bereich, in dem dem Blut innerhalb der Fasern ein zu großer Anteil an Plasmawasser entzogen wird. Durch die stärkere Aufkonzentration an Blutzellen und Proteinen wird zudem ein Verstopfen des Dialysators 6 hervorgerufen. In diesem Fall ist also der Blutfluss die beschränkende Größe.

Der Dialysator 6 sollte vorzugsweise eine Packungsdichte von mehr als 50 % aufweisen, damit ein ausreichend hoher Flusswiderstand auf der Dialysierflüssigkeitsseite existiert. In diesem Fall kann der Druckabfall durch eine Dialysierflüssigkeitsflussänderung in genügend großem Ausmaß geregelt werden.

In Fig. 4 ist ein Druckdiagramm aufgetragen, bei dem der Druck auf der Ordinate dargestellt ist. Auf der Abszisse ist die Länge des Dialysators oder die jeweilige Position im Dialysator aufgetragen. Der Druckverlauf des Blutes ist mit einer durchgezogenen Linie aufgetragen, wobei beim Eintritt in den Dialysator der Druck P1 herrscht und auf der Blut-Austrittsseite des Dialysators 6 der niedrigere Druck P2 vorhanden ist. Der Druckverlauf der im Gegenstrom strömenden Dialysierflüssigkeit ist in Fig. 4 mit einer punktierten Linie ausgehend von dem an der Dialysierflüssigkeitsaustrittsseite auftretenden, niedrigeren Druck P4 bis zu dem an der Dialysierflüssigkeitseintrittsseite auftretenden, höheren Druck P3 wiedergegeben. Die beiden Drucklinien schneiden sich bei einem Punkt Pi. Vor diesem Schnittpunkt ist der Druck auf der Blutseite größer als derjenige auf der Dialysierflüssigkeitsseite. Rechts von dem Druck Pi ist dieser Sachverhalt umgekehrt. Damit ergibt sich das in Fig. 4 dargestellte Druckkreuz, wobei eine linearisierte Berechnung unterstellt und durchgeführt wird. Auf dieser Basis lässt sich die jeweils erreichte interne Filtration während der Behandlung messen und erfassen und damit gewissermaßen "online" verarbeiten und ausgeben. In Fig. 4 ist der links von Pi liegende Bereich zwischen den beiden Drucklinien auf der Blutseite und Dialysierflüssigkeitsseite mit dem Zeichen "+" versehen, um anzugeben, dass in diesem Bereich der Druck des Blutes größer ist als derjenige der Dialysierflüssigkeit. In dem Bereich zwischen den beiden Drucklinien rechts von dem Schnittpunk Pi ist ein "-" aufgetragen, um zu verdeutlichen, dass hier der Druck auf der Blutseite niedriger ist als derjenige auf der Dialysierflüssigkeitsseite.

Anhand des in Fig. 4 gezeigten Druckkreuzes, das aus dem Blutdruck und Dialysierflüssigkeitsdruck gebildet ist, lässt sich der mittlere Transmembrandruck für die Filtration aus den Drücken P1, P2, P3, P4, Pi berechnen. Bei einem oder mehreren Ausführungsbeispielen werden P1, P2, P3 gemessen und P4 und Pi berechnet. P4 hängt vom Druckabfall auf der Dialysierflüssigkeitsseite ab und ist unter Kenntnis des Flusses und des Dialysators bzw. der Dialysatorgeometrie berechenbar. Sind der Fluss und der Dialysator 6 bekannt, kann auch eine hinterlegte Tabelle verwendet werden, um den Druckabfall anzugeben. Alternativ ist natürlich auch eine Messung mit einem zusätzlichen Drucksensor möglich.

Die Einbeziehung von online-Messungen der Drücke ist vorteilhaft, da sich diese während einer Therapie ändern können und damit folglich auch das Druckkreuz. D.h., das Druckkreuz wird für jeden Zeitpunkt bzw. für bestimmte diskrete Zeitpunkte während der Therapie neu berechnet.

Verschließen sich beispielsweise Fasern durch lokale Koagulation, auch Clotting genannt, so steigt der Druckabfall von P1 zu P2, wobei sich durch die Interaktion der Blutbestandteile mit der Membran eine sogenannte Sekundärmembran bildet. Weiterhin fällt typischerweise der dialysierflüssigkeitsseitige Druck ab, um die Ultrafiltrationsrate aufrecht zu halten. Der Druckabfall auf der Dialysierflüssigkeitsseite ändert sich nicht. Somit reicht dort weiterhin ein Druckmesspunkt, der andere kann ermittelt werden. Entscheidend für die interne Filtration sind aber nicht die absoluten Drücke, sondern die relativen von Blut- zur Dialysierflüssigkeitsseite, d. h. der Transmembrandruck. Dieser Druckgradient sorgt für den Flüssigkeitsfluss über die Membran.

Da der Transmembrandruck für die Filtration positiv definiert ist, ist dieser Bereich mit einem "+" gekennzeichnet. Die Formel lautet

$$TMP_+ = \frac{P1 + Pi}{2} - \frac{Pi + P4}{2}$$

**Gleichung 1**

Für den Fall eines höheren Dialysierflüssigkeitsdrucks ist der Transmembrandruck negativ definiert. Die Formel lautet:

$$TMP_- = \frac{P2 + Pi}{2} - \frac{Pi + P3}{2}$$

**Gleichung 2**

Hieraus lässt sich nun bei einem oder mehreren Ausführungsbeispielen unter Kenntnis des Ultrafiltrationskoeffizienten, dem Produkt aus Filteroberfläche und Permeabilität ($K_{UF}=A*p$), der konvektive Fluss oder auch "die Filtration" berechnen.

$K_{UF+}$ stellt hierbei den Ultrafiltrationskoeffizienten im Bereich des positiven Transmembrandrucks dar.

$$Q_+ = K_{UF+} * TMP_+$$

**Gleichung 2**

Gleiches gilt für Backfiltration im Bereich des negativen Transmembrandrucks.

$$Q_- = K_{UF_-} * TMP_-$$

**Gleich**

**ung 4**

$K_{UF+}$ und $K_{UF-}$ sind bei einem oder mehreren Ausführungsbeispielen in einer Tabelle für den jeweiligen Dialysator 6 hinterlegt.

Da sich die Filtration von der Backfiltration um den Weight Loss, also die dem Patienten zur Entwässerung effektiv entzogene Flüssigkeit, unterscheidet, gilt

$$\left| Q_+ \right| = \left| Q_- \right| + \left| Q_{WL} \right|$$

**Gleich**

**ung 5**

Alle angegebenen Flussgrößen können in verschiedenen Einheiten angegeben werden. Typisch: ml/min, aber auch möglich sind z.B.: ml/h, l/h, ml/Therapie, l/Therapie. Dieses Prinzip ist generell für HD Therapien gedacht, kann aber auch im Falle einer HDF oder einer single-needle Therapie verwendet werden. Hierbei steht HD für Hämodialyse und HDF für Hämodiafiltration.

Im Folgenden wird eine Berechnung unter Einbeziehung weiterer Parameter beschrieben.

Durch die interne Filtration ändert sich beispielsweise der Hämatokrit (Hct) innerhalb des Dialysators. Dies hat direkten Einfluss auf die Viskosität (A. Wüpper, D. Woermann, F. Dellanna, and C. A. Baldamus : Retrofiltration rates in high-flux hollow fiber hemodialyzers: analysis of clinical data - Journal of Membrane Science, 121:109 - 116, 1996)

$$\eta_B = \eta_P(1 + 2,5 Hct + 7,35 Hct^2)$$

**Gleich**

**ung 6**

Hierbei bezeichnet $\eta_B$ die Viskosität des Blutes und $\eta_P$ die des Blutplasmas.

Letztere kann zum Beispiel gemäß der nachfolgenden Gleichung berechnet werden:

$$\frac{\eta_P}{\eta_W} = 1 + \left\{ \frac{\eta_{P,R}}{\eta_W} - 1 \right\} \cdot \left\{ \frac{c_P}{c_{P,R}} \right\}$$

**Gleich**

**ung 7**

Hierbei ist $\eta_{P,R}$ der Referenzwert der Plasmaviskosität, $c_{P,R}$ die Plasmaproteinkonzentration der Referenzlösung, $\eta_W$ die Viskosität von Wasser, $\eta_P$ die des Plasmas, $c_P$ die aktuelle Plasmaproteinkonzentration und Hct der Hämatokrit. Die Referenzwerte $\eta_{P,R}$ und $c_{P,R}$ sind als Standard hinterlegt, können aber auch patientenspezifisch aufgenommen und dann hinterlegt werden.

Zusätzlich ändern sich die Flüsse auf der Blut- und der Dialysierflüssigkeitsseite in Abhängigkeit von der lokalen Filtration.

Es lässt sich somit ein Verlauf der Drücke innerhalb des Dialysators 6 berechnen, der von der Linearität abweicht und die eigentlichen Prozesse innerhalb des Dialysators genauer abbildet. Dies ist in Fig. 5 gezeigt, für die die Erläuterungen zu Fig. 4 gleichfalls zutreffen. Der Blutdruckverlauf ist hier nicht linear.

Auch unter diesen Bedingungen lässt sich der mittlere Transmembrandruck für Filtration und für Backfiltration unter Berücksichtigung der Kurvenform bestimmen und es lassen sich die jeweiligen Flüsse berechnen.

Zusätzlich kann anhand des durch einen Hämatokritsensor erfassten Hämatokrit-Wertes, Hct Wertes, der onkotische Druck, d.h. der durch die Kolloide einer Lösung bewirkte Anteil am osmotischen Druck, berechnet werden, der an den Messpunkten außerhalb des Dialysators 6 nicht erfasst wird. Er berechnet sich nach (E. M. Landis and J. R. Pappenheimer: Exchange of substances through the capillary walls - Handbook of Physiology - Section 2: Circulation, 11:961 - 1034, 1963):

$$\Pi = 2,1 \cdot c_P + 0,16 \cdot c_P^2 + 0,009 \cdot c_P^3$$

Gleich

ung 8

Dieser Sachverhalt ist bei dem Kurvenverlauf gemäß Fig. 4 zugrunde gelegt.

Typischerweise wird der onkotische Druck von dem blutseitigen Druckverlauf subtrahiert. Es ist aber auch eine Addition auf der Dialysierflüssigkeitsseite möglich.

Alle Eingangswerte für Druck, Hämatokrit, Plasmaproteinkonzentration, die Referenzwerte und weitere, hier nicht erwähnte verwendbare Größen können messtechnisch erhoben werden oder aus einer auf oder in der Maschine hinterlegten Tabelle übertragen, bzw. berechnet werden .

Bei einem oder mehreren Ausführungsbeispielen der Erfindung erfolgt eine Berechnung des Verlaufes eines Parameters, beispielsweise der Druckverläufe oder auch der internen Filtration oder sonstiger abhängiger Größen innerhalb des Dialysators 6.

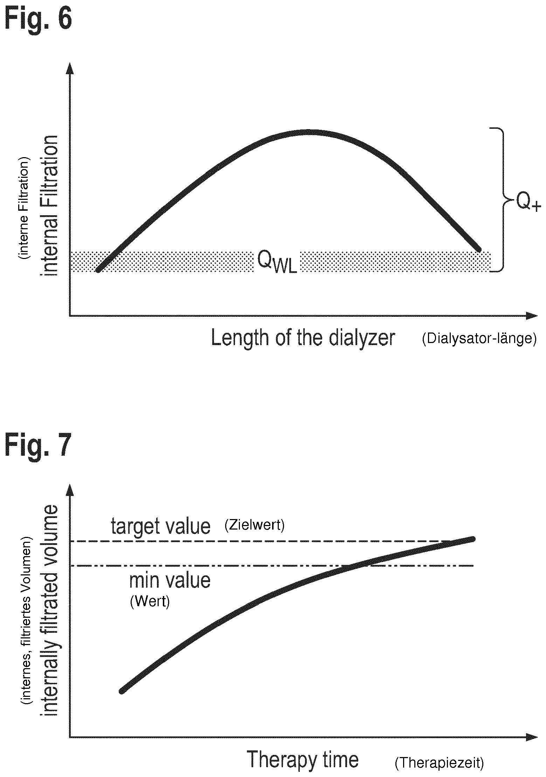

In Fig. 6 ist ein Beispiel für diese Berechnung des Verlaufs innerhalb des Dialysators 6 aufgetragen. Hier ist als Beispiel die interne Filtration herangezogen. Anhand der Druckverläufe, die durch die Drucksensoren 5a bis 5d (Fig. 1) ermittelt werden, lässt sich ein Profil der internen Filtration oder auch ein Profil jeder abhängigen Größe wie etwa beispielsweise des Blutflusses oder Dialysierflüssigkeitsflusses, des Hämatokrits, der Blutviskosität oder anderer Parameter oder einer Kombination dieser Parameter oder auch die Gesamtheit aller dieser Parameter erstellen.

Bei dem in Fig. 6 gezeigten Beispiel ist auf der Abszisse wiederum die Länge des Dialysators, d. h. die jeweilige Position innerhalb des Dialysators 6, aufgetragen, während auf der Ordinate die interne Filtration angegeben ist. Wie ersichtlich, ist die interne Filtration im Inneren des Dialysators 6 erwartungsgemäß größer und verläuft bogenförmig gekrümmt mit einem etwas oberhalb der Mitte des Dialysators 6 auftretenden Maximum, während das Minimum am linken Dialysatorende, d. h. am Dialysatoreinlass auftritt. Die gesamte Filtrationsspanne ausgehend von dem links auftretenden Filtrationsminimum bis zu dem in der rechten Hälfte auftretenden Filtrationsmaximum ist am rechten Ende des Schaubilds der Fig. 6 mit "$Q_+$" aufgetragen. Mit $Q_{WL}$ ist das Band zwischen dem einlassseitigen und dem dialysatorauslassseitigen Filtrationswert veranschaulicht.

Bei einem oder mehreren Ausführungsbeispielen der Erfindung ist eine Anpassung der berechneten Filtration möglich. Ändern sich beispielsweise im Verlauf der Therapie die Drücke, kann dies über die Drucksensoren 5a bis 5d erfasst werden. Diese Änderung von einem oder mehreren Drücken kann dann in der Berechnung des jeweiligen Parameters, beispielsweise des Verlaufes der Filtration innerhalb des Dialysators 6, berücksichtigt werden.

Ändert sich beispielsweise der Ultrafiltrationskoeffizient aufgrund einer Wechselwirkung der Blutbestandteile mit der Membran des Dialysators 6, lässt sich auch diese Änderung des Ultrafiltrationskoeffizienten ermitteln und berücksichtigen. Durch eine Änderung der Flussrate, mit der dem gerade behandelten Patienten Flüssigkeit entzogen wird, beispielsweise der Weight-Loss-Rate oder Ultrafiltrationsrate, kann eine sich hierbei einstellende Änderung des Transmembrandrucks TMP beobachtet und erfasst werden. Hieraus lässt sich dann der neue Ultrafiltrationskoeffizient analog zu den vorstehend angegebenen Gleichungen 3 und 4 bestimmen und hieraus die neue Filtration erfassen und festlegen. Bei konstanter Ultrafiltration kann eine Änderung des Transmembrandrucks TMPs beobachtet und zur Korrektur des Ultrafiltrationskoeffizienten $K_{UF}$ verwendet werden. Hierbei wird nicht direkt der TMP, sondern vielmehr der $K_{UF}$ benötigt.

Hierbei kann in gleicher Weise auch ein neuer Eingangshämatokrit oder eine andere beliebige Variable, die während der Therapie gemessen wird, in die Berechnung einbezogen werden. Hierdurch lässt sich die Berechnung weiter verfeinern oder auch eine andere Größe ermitteln. Unter Berücksichtigung eines Siebkoeffizienten für bestimmte Moleküle, der beispielsweise aus einem Datenblatt entnommen werden kann, lässt sich zudem eine konvektive Clearance für verschiedene Substanzen angeben.

Bei einem oder mehreren Ausführungsbeispielen der Erfindung erfolgt eine Anpassung der internen Filtration während der Patientenbehandlung, das heißt "online". Hierzu wird vorzugsweise die Fläche zwischen den Druckkurven, siehe z. B. Fign. 2, 3, 4 beeinflusst. Dadurch lässt sich die interne Filtration regeln. Zur Beeinflussung und zur Bestimmung des Verhaltens der Druckkurven erfolgt vorzugsweise in guter Näherung eine Orientierung an dem Gesetz von Hagen-Poiseuille, gemäß dem der Druckabfall proportional zum jeweiligen Fluss ist.

Ein größerer Druckabfall vergrößert hierbei die Flächen zwischen den Druckkurven auf der Blut- und Dialysierflüssigkeitsseite und somit die Werte TMP+, TMP-, die in den Fign. 4 und 5 jeweils mit den Zeichen "+", "-" symbolisiert sind. Bei einem oder mehreren Ausführungsbeispielen der Erfindung werden somit der Blutfluss und/oder der Dialysierflüssigkeitsfluss gesteuert oder geregelt, um beispielsweise einen Sollwert zu erreichen oder einen Mindestwert zu erzielen.

Bei einem oder mehreren Ausführungsbeispielen der Erfindung kann beispielsweise durch Schließen einer venösen Absperrklemme im Blutrückfluss zum Patienten die Bilanzierung kurzzeitig gestört werden und somit eine höhere Flussrate an Filtration erzeugt werden, die durch eine Anpassung des dialysierflüssigkeitsseitigen Druckes ausgeglichen wird. Hierzu kann beispielsweise die Dialysierflüssigkeitspumpe 9 kurzzeitig verstärkt angesteuert werden, wobei der hierdurch erzeugte erhöhte Druck über den Drucksensor 5c erfasst wird. Weiterhin kann beispielsweise ein wiederholtes Schließen, z. B. über die venöse Absperrklemme durchgeführt werden, wodurch eine Pulsation erzeugt wird. Alternativ oder zusätzlich hierzu kann auch ein Ventil im Dialysierflüssigkeitsausfluss (links am Auslass der Ultrafiltrationspumpe 7 gemäß Fig. 1) kurzzeitig einmal oder vorzugsweise mehrfach geschlossen werden, um die Bilanzierung kurzzeitig zu stören.

Ferner kann das Druckverhältnis durch Anlegen eines äußeren Unterdrucks beeinflusst werden. Hierzu kann beispielsweise die Ultrafiltrationspumpe 7 verwendet werden, indem diese beispielsweise verstärkt angesteuert wird, um hierdurch einen Unterdruck in der Dialysierflüssigkeitskammer 6b zu generieren

Bei einem oder mehreren Ausführungsbeispielen der Erfindung kann zusätzlich oder alternativ zu den vorstehend angegebenen Maßnahmen auch die Backfiltration als Regelgröße verwendet werden.

Bei einem oder mehreren Ausführungsbeispielen der Erfindung kann beispielsweise auf der Anzeige 1 gemäß Figur 1 der aktuelle Wert der Filtration ausgegeben werden. Hierbei kann beispielsweise der in Fig. 7 dargestellte Kurvenverlauf auf dem Monitor der Anzeige 7 dargestellt werden, der zu jedem Zeitpunkt der Therapie zeigt, welches Volumen schon filtriert wurde.

In Figur 7 ist auf der Abszisse die Therapiezeit, also die seit Therapiebeginn jeweils verstrichene Zeit aufgetragen, während auf der Ordinate das jeweils intern filtrierte Volumen aufgetragen ist. Der kontinuierlich ansteigende Kurvenzug veranschaulicht das filtrierte Volumen. Ferner sind in Fig. 7 zwei horizontale, gestrichelte Linien aufgetragen, von denen die obere den optimal zu erreichenden Zielwert ("target value") veranschaulicht, der den vollen Therapieerfolg signalisiert. Die darunter liegende doppelpunktierte Strichlinie repräsentiert den minimalen Wert ("min value"), der für eine ausreichende Therapie minimal erreicht werden sollte. Anhand der Kurvendarstellung gemäß Fig. 7 lässt sich auf der Anzeige 1 unmittelbar erkennen, wie weit die Therapie bereits vorgeschritten ist und welche Therapiedauer noch zu erwarten ist. Weiterhin lassen sich eventuelle unerwartete Therapieverläufe sofort erkennen und geeignete Gegenmaßnahmen ergreifen.

Auf der Anzeige 1 lässt sich alternativ oder zusätzlich auch die Veränderung des Flusses (als Volumen pro Zeiteinheit) darstellen.

Der bisherige, auf der Anzeige 1 ersichtliche oder in der Vorrichtung gespeicherte Therapieverlauf ab dem Beginn der Behandlung bis zum aktuellen Zeitpunkt lässt sich auch zur Berechnung und Vorhersage des weiteren Verlaufs auswerten. Damit kann auch der erwartete Therapieverlauf ab dem aktuellen Zeitpunkt der Behandlung bis zu deren Ende als Vorhersage des weiteren Verlaufs dargestellt werden. Hierdurch wird dem Anwender angezeigt, ob und wann das gewünschte Ziel zum Ende der Therapie erreicht wird.

Zur Vorhersage des Therapieverlaufs können zusätzlich auch registrierte oder in anderer Weise gespeicherte Daten aus vorangegangenen Therapien verwendet werden. Auf der Basis solcher Daten kann mittels geeigneter Auswertungs- und Prädiktionsprogramme auf den zu erwartenden zukünftigen Verlauf geschlossen werden.

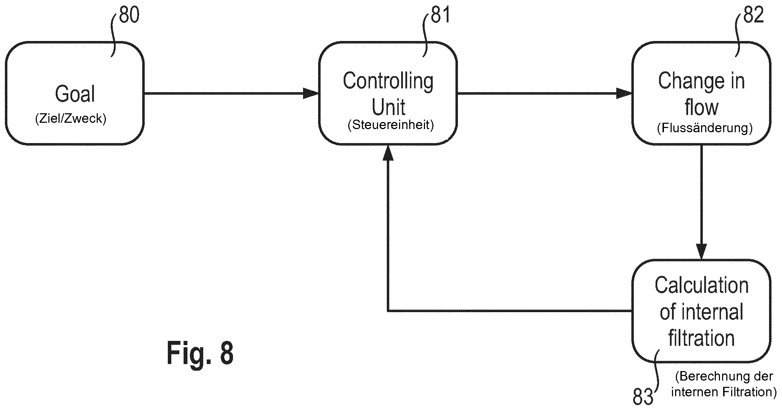

In Fig. 8 ist in Form eines Blockschaltbilds ein Ausführungsbeispiel eines Regelkreises und einer Regelung dargestellt, die bei Ausführungsbeispielen der erfindungsgemäßen Vorrichtung eingesetzt werden können.

Über eine Eingabe 80, die als konstruktive Einrichtung realisiert sein kann, wird der jeweils gewünschte Zielwert eingegeben, siehe z. B. den in Fig. 7 eingetragenen Zielwert "target value". Eine Steuereinheit 81 vergleicht den aktuellen Wert der Filtration, wie er beispielsweise anhand einer Berechnung der internen Filtration ermittelt worden ist, mit dem Zielwert gemäß der Eingabe 80. Die Steuereinheit 81 kann beispielsweise dem Regler 2 gemäß Fig. 1 entsprechen. Der aktuelle Wert der internen Filtration wird durch eine Berechnungseinrichtung 83 bzw. einen entsprechenden Berechnungsschritt ermittelt, die die jeweilige interne Filtration in ihrem Wert beispielsweise auf der Basis der vorstehenden Ausführungen ermittelt. Die Steuereinrichtung 1 passt bei Abweichungen zwischen dem aktuellen Wert der internen Filtration, wie er durch die Einrichtung 83 bzw. den entsprechenden Berechnungsschritt ermittelt worden ist, von dem Zielwert gemäß Eingabe 80 beispielsweise den Blutfluss und/oder den Dialysierflüssigkeitsfluss entsprechend an, indem sie eine Änderung der Strömung hervorruft. Dies ist durch den Block 82 veranschaulicht. Diese Änderung des Blutflusses und/oder Dialysierflüssigkeitsflusses kann beispielsweise gemäß den vorstehenden Ausführungen erfolgen, indem die Blutpumpe 3, die Dialysierflüssigkeitspumpe 9 und/oder die Ultrafiltrationspumpe in ihrer Ansteuerung verändert werden.

Die über die Einrichtung 80 bzw. den entsprechenden Schritt eingegebene Zielgrösse, das heißt der Zielwert, kann beispielsweise eine Flussrate in ml/min, ml/h, ml/Therapie, ... usw. sein.

Mit einem oder mehreren Ausführungsbeispielen der erfindungsgemäßen Vorrichtung lässt sich somit eine Regelung auf die interne Filtration oder die Backfiltration erreichen.

Mit den Ausführungsbeispielen der Erfindung lässt sich folglich eine Kenntnis der internen Filtration und damit der konvektiven Reinigungsleistung während der Therapie, also der Hämodialyse, Hämodiafiltration oder dergleichen, erreichen. Dies erlaubt weiterhin eine Regelung der konvektiven und damit der mittelmolekularen Clearance.

Im Folgenden wird ein konkretes Ausführungsbeispiel zahlenmäßig beschrieben, ohne dass jedoch hierin eine Einschränkung auf nur solche Zahlenwerte zu sehen ist.

Gemäß einem Ausführungsbeispiel der Erfindung wählt der Anwender beim Vorbereiten der Dialysebehandlung z. B. eine untere Grenze für das durch interne Filtration zu erzeugende Volumen von 12 l bis zum Ende der Therapie aus.

Bei Therapiestart werden der Hämatokrit am Eingang des Dialysators 6 und die Drücke auf der Blut- sowie Dialysierflüssigkeitsseite gemessen. Hieraus wird der Verlauf der Druckkurven innerhalb des Dialysators 6 unter Berücksichtigung des onkotischen Drucks berechnet. Bei einem gemessenen Hämatokrit von z. B. 32 % liegt die Permeabilität für Filtration bei 90 ml/(h mmHg m$^2$) und die für Backfiltration bei 265 ml/(h mmHg m$^2$).

Die aktuelle interne Filtrationsrate wird berechnet und z. B. mit 56 ml/min angegeben.

Während der ersten Behandlungsstunde verändern sich die Dialysatoreigenschaften sowie die Drücke am Dialysator 6. Die neu berechnete interne Filtrationsrate liegt nur noch bei z. B. 48 ml/min. Aufgrund der fallenden Tendenz ist die Vorhersage für das erreichte, intern filtrierte Volumen nun 11 l.

Dem Anwender wird z. B. über die Anzeige 1 oder eine akustische Ausgabe empfohlen, den Dialysierflüssigkeitsfluss von 400 ml/min auf 500 ml/min zu erhöhen, um über den so erzeugten größeren Druckabfall auf der Dialysierflüssigkeitsseite größere lokal auftretende Transmembrandrücke TMP+ und TMP- zu erzeugen und dadurch die interne Filtrationsrate wieder zu erhöhen.

Nach Anpassung des Dialysierflüssigkeitsflusses wird die interne Filtrationsrate neu berechnet und ergibt sich z. B. zu 52 ml/min.

Während der nächsten drei Behandlungsstunden treten keine weiteren Veränderungen auf und es wird ein intern filtriertes Volumen von 12,4 l erzielt.

Gemäß einem weiteren Beispiel wählt der Anwender beim Vorbereiten der Dialysebehandlung eine untere Grenze für die online Messung der mittelmolekularen Clearance von z. B. 70 ml/min aus. Die Vorrichtung setzt bei diesem Ausführungsbeispiel einen Wert von z.B. 75 ml/min als Warngrenze für den Erhalt der gewünschten Clearance.

Nach Therapiestart wird die erste Messung durchgeführt und es ergeben sich z. B. 83 ml/min.

Nach einer Therapiedauer von 90 min wird der Anwender per Meldung auf dem Monitor 1 der Dialysemaschine darauf hingewiesen, dass die mittelmolekulare Clearance nun bei 75 ml/min liegt und voraussichtlich weiter fallen wird. Es wird z. B. optisch über die Anzeige oder akustisch empfohlen, den Blutfluss von 300 ml/min auf 330 ml/min zu erhöhen. Durch die Flussanpassung liegt der nächste gemessene Wert für die mittelmolekulare Clearance bei 82 ml/min.

Bis zum Ende der Therapie liegt der Wert konstant über der Warngrenze von 75 ml/min.

Durch die online Bestimmung der internen Filtration kann der Verlauf derselben über die gesamte Therapie angegeben werden, und es können Behandlungsparameter angepasst werden, um diese Filtration zu erhalten.

**Patentansprüche**

1. Vorrichtung zum Reinigen von Blut,
   mit einer Reinigungseinrichtung, vorzugsweise in Form eines Dialysators (6), die von dem Blut durchströmbar ist, und einer Ermittlungseinrichtung zur Ermittlung einer internen Konvektion in der Reinigungseinrichtung auf der Grundlage von Druckunterschieden an der Reinigungseinrichtung,
   wobei die Vorrichtung dazu ausgelegt ist,
   einen mittleren Transmembrandruck für die Filtration anhand von ermittelten Drücken zu berechnen,
   ein Druckkreuz aus dem Blutdruckverlauf und einem Druckverlauf einer Reinigungsflüssigkeit, vorzugsweise Dialysierflüssigkeit, zu ermitteln, und
   die interne Konvektion auf der Basis des Transmembrandrucks und gegebenenfalls zusätzlich unter Berücksichtigung eines Ultrafiltrationskoeffizienten als Produkt aus einer Filteroberfläche und einer Permeabilität zu berechnen,
   wobei der Druckverlauf des Blutes mit einer ersten Drucklinie auftragbar ist und der Druckverlauf der im Gegenstrom strömenden Reinigungsflüssigkeit mit einer zweiten Drucklinie wiedergebbar ist und sich die beiden Drucklinien an einem Punkt (Pi) schneiden, woraus sich das Druckkreuz ergibt, **dadurch gekennzeichnet dass** die Vorrichtung eine Regeleinrichtung (2) aufweist, die dazu ausgelegt ist, die ermittelte konvektive Filtration zu regeln.

2. Vorrichtung nach Anspruch 1, bei der die Blutreinigung eine Hämodialyse oder eine Hämodiafiltration ist, mit Drucksensoren zur Messung der Drücke des Bluts am Eingang und/oder am Ausgang der Reinigungseinrichtung.

**3.** Vorrichtung nach Anspruch 1 oder 2, mit mindestens einem Drucksensor (4) zur Erfassung des Drucks einer Reinigungsflüssigkeit oder Dialysierflüssigkeit am Eingang und/oder am Ausgang der Reinigungseinrichtung.

**4.** Vorrichtung nach einem der Ansprüche 1 bis 3, die dazu ausgelegt ist, die Flüsse einer Reinigungsflüssigkeit und/oder des Bluts zu ermitteln und basierend hierauf die Druckunterschiede rechnerisch oder anhand vorab hinterlegter Dialysatorspezifischer Kennlinien oder Wertetabellen zu bestimmen.

**5.** Vorrichtung nach einem der Ansprüche 1 bis 4, bei der die Ermittlungseinrichtung zusätzlich eine Hämatokritbestimmung oder Hämatokritvorgabe und/oder eine Ermittlung oder Vorgabe einer Plasmaviskosität oder einer Plasmaproteinkonzentration bewirkt.

**6.** Vorrichtung nach einem der Ansprüche 1 bis 5, wobei der Reinigungsflüssigkeitsdruckverlauf ein Dialysierflüssigkeitsdruckverlauf ist.

**7.** Vorrichtung nach einem der Ansprüche 1 bis 6, bei der die Regeleinrichtung dazu ausgelegt ist, einen ermittelten Wert der internen Konvektion mit einem vorgegebenen Sollwert zu vergleichen und den Blutfluss des zu reinigenden Bluts und/oder den Fluss einer Reinigungsflüssigkeit wie etwa einer Dialysierflüssigkeit zu regeln.

**8.** Vorrichtung nach einem der Ansprüche 1 bis 7, **gekennzeichnet durch** eine Anzeige zum Anzeigen der ermittelten internen Konvektion.

**Claims**

**1.** An apparatus for the purification of blood, comprising
a purification device through which blood can flow, preferably in the form of a dialyzer, and
a determination device for determining an internal convection in the purification device on the basis of pressure differences in the purification device, wherein said apparatus is configured
to calculate a median transmembrane pressure for the filtration with the aid of determined pressures,
to determine a pressure intersection made up of the blood pressure profile and a pressure profile of a cleaning fluid, preferably a dialysis fluid, and
to calculate the internal convection on the basis of the transmembrane pressure and possibly additionally in consideration of an ultrafiltration coefficient as a product of a filter surface and a permeability, wherein
the pressure curve of the blood is representable with a first pressure line, and the pressure curve of the dialysis fluid flowing in countercurrent is representable with a second pressure line, said two pressure lines intersecting at a point (Pi), from which said pressure follows, **characterized in that**
said apparatus comprises a control means (2) which is designed to control the determined convective filtration..

**2.** The apparatus according to claim 1, in which the blood purification is a hemodialysis or a hemodiafiltration, comprising pressure sensors for measuring the pressures of the blood at the input and/or output of the purification device.

**3.** The apparatus according to claim 1 or 2, comprising at least one pressure sensor for detecting the pressure of a cleaning fluid or dialysis fluid at the input and/or output of the purification device.

**4.** The apparatus according to any of the claims 1 to 3, which is designed to determine the flows of a cleaning fluid and/or of the blood and ascertain the pressure differences on the basis thereof by calculation or with the aid of characteristic curves or value tables which are specific to the dialyzer and have been stored in advance.

**5.** The apparatus according to any of the claims 1 to 4, in which the determination device additionally effects a hematocrit determination or a hematocrit presetting and/or a determination or presetting of a plasma viscosity or of a plasma protein concentration.

**6.** The apparatus according to any of the claims 1 to 5, in which said cleaning fluid pressure profile is a dialysis fluid pressure profile.

**7.** The apparatus according to one of the claims 1 to 6, in which the control unit is designed to compare a determined value of the internal convection with a predefined target value and to control the flow of the blood to be cleaned and/or the flow of a cleaning fluid such as a dialysis fluid.

8. The apparatus according to any of the claims 1 to 7, **characterized by** a display for displaying the determined internal convection.

**Revendications**

1. Dispositif de purification de sang,
   avec un dispositif de purification, de préférence sous forme de dialyseur (6), qui peut être traversé par le sang, et un dispositif de détermination pour la détermination d'une convection interne dans le dispositif de purification sur la base de différences de pression au niveau du dispositif de purification,
   dans lequel le dispositif est conçu pour
   calculer une pression transmembranaire moyenne pour la filtration à l'aide de pressions déterminées,
   pour déterminer une croix de pression à partir de la courbe de pression du sang et d'une courbe de pression d'un fluide de purification, de préférence un fluide de dialyse, et
   pour calculer la convection interne sur la base de la pression transmembranaire et le cas échéant en plus en tenant compte d'un coefficient d'ultrafiltration comme produit d'une surface de filtration et d'une perméabilité,
   dans lequel la courbe de pression du sang peut être appliquée avec une première ligne de pression et la courbe de pression du fluide de purification circulant à contre-courant peut être représentée avec une deuxième ligne de pression et les deux lignes de pression se coupent en un point (Pi), ayant pour résultat la croix de pression, **caractérisé en ce que** le dispositif présente un dispositif de régulation (2), qui est conçu pour réguler la filtration convective déterminée.

2. Dispositif selon la revendication 1, dans lequel la purification du sang est une hémodialyse ou une hémodiafiltration, avec des capteurs de pression pour la mesure des pressions du sang à l'entrée et/ou à la sortie du dispositif de purification.

3. Dispositif selon la revendication 1 ou 2, avec au moins un capteur de pression (4) pour la détection de la pression d'un fluide de purification ou fluide de dialyse à l'entrée et/ou à la sortie du dispositif de purification.

4. Dispositif selon l'une quelconque des revendications 1 à 3, conçu pour déterminer les flux d'un fluide de purification et/ou du sang et pour déterminer sur cette base les différences de pression par calcul ou à l'aide de courbes caractéristiques ou de tableaux de valeurs spécifiques au dialyseur enregistrés au préalable.

5. Dispositif selon l'une quelconque des revendications 1 à 4, dans lequel le dispositif de détermination entraîne en outre une détermination d'hématocrite ou une prescription d'hématocrite et/ou une détermination ou prescription d'une viscosité plasmatique ou d'une concentration de protéines plasmatiques.

6. Dispositif selon l'une quelconque des revendications 1 à 5, dans lequel la courbe de pression de fluide de purification est une courbe de pression de fluide de dialyse.

7. Dispositif selon l'une quelconque des revendications 1 à 6, dans lequel le dispositif de régulation est conçu pour comparer une valeur déterminée de la convection interne avec une valeur de consigne prescrite et de réguler le flux sanguin du sang à purifier et/ou le flux d'un fluide de purification comme un fluide de dialyse.

8. Dispositif selon l'une quelconque des revendications 1 à 7, **caractérisé par** un affichage pour l'affichage de la convection interne déterminée.

# Fig. 1

Graph axis labels: internally filtrated volume (internes, filtriertes Volumen); target value (Zielwert); min value (Wert); Therapy time (Therapiezeit)

# Fig. 2

Graph axis labels: internal filtration (interne Filtration); blood flow (Blutfluss)

# Fig. 3

internal filtration (interne Filtration)

dialysis fluid flow (Dialysierflüssigkeitsfluss)

# Fig. 4

Pressure (Druck)

P1

P4

+

Pi

—

P3

P2

Length of the dialyzer (Dialysator-länge)

# Fig. 5

Pressure (Druck)

P1

P4

+

Pi

—

P3

P2

Length of the dialyzer (Dialysator-länge)

## Fig. 6

## Fig. 7

82 — Change in flow (Flussänderung)

83 — Calculation of internal filtration (Berechnung der internen Filtration)

81 — Controlling Unit (Steuereinheit)

80 — Goal (Ziel/Zweck)

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0240101 A2
- EP 1867353 A
- US 4381999 A
- DE 102007009208 A1

**In der Beschreibung aufgeführte Nicht-Patentliteratur**

- **A. WÜPPER ; D. WOERMANN ; F. DELLANNA ; C. A. BALDAMUS.** Retrofiltration rates in high-flux hollow fiber hemodialyzers: analysis of clinical data. *Journal of Membrane Science,* 1996, vol. 121, 109-116
- Exchange of substances through the capillary walls. **E. M. LANDIS ; J. R. PAPPENHEIMER.** Handbook of Physiology - Section 2: Circulation. 1963, vol. 11, 961-1034